**Europäisches Patentamt**

**European Patent Office**

**Office européen des brevets**

(19)

(11) Veröffentlichungsnummer: **0 042 083**
**B1**

(12) # EUROPÄISCHE PATENTSCHRIFT

(45) Veröffentlichungstag der Patentschrift:
**16.05.84**

(51) Int. Cl.³: **A 61 L 15/06**, C 09 J 7/02

(21) Anmeldenummer: **81103991.6**

(22) Anmeldetag: **25.05.81**

(54) **Wasserfester Klebestreifen für medizinische Zwecke.**

(30) Priorität: **16.06.80 DE 3022605**

(43) Veröffentlichungstag der Anmeldung:
**23.12.81 Patentblatt 81/51**

(45) Bekanntmachung des Hinweises auf die Patenterteilung:
**16.05.84 Patentblatt 84/20**

(84) Benannte Vertragsstaaten:
**AT BE CH DE FR GB IT LI NL SE**

(56) Entgegenhaltungen:
**FR - A - 2 384 503**

(73) Patentinhaber: **Beiersdorf Aktiengesellschaft,
Unnastrasse 48, D-2000 Hamburg 20 (DE)**

(72) Erfinder: **Müller, Heinz, Dr. Dipl.-Chem., Wittkoppel 29,
D-2000 Hamburg 54 (DE)**
Erfinder: **Szonn, Bodo, Chem.-Ing. grad., Segeberger
Strasse 42, D-2359 Kisdorf (DE)**
Erfinder: **Jauchen, Peter, Chem.-Ing. grad.,
Frohmestrasse 64, D-2000 Hamburg 61 (DE)**
Erfinder: **Schulze, Rolf, Hoistenstrasse 16,
D-2000 Hamburg 50 (DE)**

# 0 042 083

## Wasserfester Klebestreifen für medizinische Zwecke

Die Erfindung betrifft einen neuen wasserfesten Klebestreifen für medizinische Zwecke, der aus einem einseitig mit einer Selbstklebemasse versehenen Trägerstreifen auf Basis eines porösen, mindestens in einer Richtung elastischen dünnen Faservlies besteht und auf seiner Rückseite mit einer wasserundurchlässigen Beschichtung versehen ist.

An ein hochwertiges selbstklebendes Pflaster, das möglichst vielseitig einsetzbar ist, werden in der Praxis eine ganze Reihe von Anforderungen gestellt. Es soll beispielsweise neben einer guten Klebkraft auf der Haut, guter Hautverträglichkeit sowie Luft- und Wasserdampfdurchlässigkeit, angenehmen Trageeigenschaften, rückstandsfreier und leichter Wiederablösbarkeit sowie Beständigkeit gegenüber mechanischer Beanspruchung insbesondere auch eine gute Wasserfestigkeit aufweisen und die Wunde vor von außen einwirkender Feuchtigkeit schützen, ohne die Hautatmung zu behindern.

Die seit langem bekannten Pflaster aus Baumwoll- oder Zellwollgewebe mit einer Klebemasse auf Kautschuk/Harz-Basis erfüllen diese Forderungen unter anderem auch in bezug auf eine gute Wasserfestigkeit nur unvollständig.

Einen ersten Fortschritt zur Erzielung einer guten Wasserbeständigkeit in Verbindung mit vorteilhaften Trageeigenschaften erzielte man durch die Verwendung von thermoplastischen Weichfolien als Trägermaterial. Da diese Folien aus Polyvinylchlorid oder Polyäthylen jedoch weitgehend wasserdampfundurchlässig sind, müssen sie mechanisch perforiert werden, um eine ausreichende Klebkraft — hierzu ist eine genügende Wasserdampfdurchlässigkeit erforderlich — auf der Haut zu erzielen. Dadurch wird aber zwangsläufig wegen der Größe der durch die mechanische Perforation erzeugten Löcher ihre Schutzwirkung gegenüber Wassereinwirkung wieder vermindert. Mikroporöse Folien weisen diesen Nachteil nicht auf, sind jedoch aus technischen Gründen mit 1—2 mm Stärke vergleichsweise dick und auftragend.

Die in neuerer Zeit entwickelten Pflaster mit einem Träger aus Vlies, die mit einer Selbstklebemasse auf Polyacrylat-Basis nach speziellen Verfahren mikroporös beschichtet sind, besitzen sowohl eine gute Klebkraft auf der Haut als auch eine ausgezeichnete Hautverträglichkeit verbunden mit einer guten Wiederablösbarkeit ohne Rückstandsbildung auf der Haut. Ferner ist die Alterungsbeständigkeit dieser Klebemassen aufgrund fehlender Doppelbindungen im Vergleich zu den Klebern auf Basis von Kautschuken hervorragend.

Die in Verbindung mit diesen Selbstklebemassen benutzten Vliese als Trägermaterialien verleihen den hergestellten Pflastern ausgezeichnete Trageeigenschaften, d. h. sie sind vor allem weich, anschmiegsam sowie luft- und wasserdampfdurchlässig. Durch die Kombination dieser beiden neuen Systeme gelang es, fast alle an ein ideales Pflaster gestellten Forderungen zu erfüllen mit Ausnahme der nach einer guten Beständigkeit bei mechanischer Belastung und Einwirkung von Wasser. Die beiden nicht gelösten Probleme waren bisher ein starker Hinderungsgrund, die Pflaster universeller einzusetzen.

Die genannten Nachteile weist auch der Wundschnellverband gemäß der FR-PS 2 384 503 auf mit einem Trägerstreifen aus einem nach dem Spunlaced-Verfahren hergestellten bindemittelfreien, porösen Polyester-Vlies, das vorzugsweise querelastisch ausgebildet ist. Ein derartiges Vlies hat zwar sehr gute Trageeigenschaften und stellt auch ein bevorzugtes Ausgangsmaterial für die Herstellung des erfindungsgemäßen Pflasters dar, es besitzt jedoch keine Wasserfestigkeit und kann deshalb nur in einem begrenzten Einsatzbereich verwendet werden.

Aufgabe der Erfindung war es deshalb, unter Beibehaltung aller guten Eigenschaften die Widerstandsfähigkeit der Vlies-Pflaster gegenüber mechanischer Belastung und der Einwirkung von Wasser wesentlich zu verbessern. Hierzu war es erforderlich, ein Beschichtungssystem zu finden, durch das die porösen Vliesträger einerseits mit einer festen und wasserundurchlässigen Schicht versehen werden, so daß die daraus hergestellten Pflaster ausreichend mechanisch stabil und wasserfest sind, und andererseits doch ausreichend mikroporös sind und damit die Hautatmung ermöglichen.

Die Lösung der Aufgabe bestand in der Erfüllung dieser beiden sich widersprechenden Forderungen, ohne einen Kompromiß zu schließen. Sie gelang im Sinn der gestellten Forderungen durch die besondere Auswahl eines hierfür geeigneten Vliesträgers in Verbindung mit einem hierfür entwickelten Spezialbeschichtungssystem.

Der erfindungemäße wasserfeste Klebestreifen für medizinische Zwecke aus einem einseitig mit einer Selbstklebemasse versehenen Trägerstreifen auf Basis eines porösen, mindestens in einer Richtung elastischen dünnen Faservlieses ist dadurch gekennzeichnet, daß das Faservlies Poren einer Größe von 0,5 bis 2 mm aufweist und auf seiner nichtklebenden Rückseite mit einer ca. 5—10 µm starken duroplastischen, wasserundurchlässigen Lackschicht versehen ist, welche mittels einer porösen, ca. 1—3 µm starken elastischen Zwischenschicht mit der Vliesoberfläche verbunden ist.

Von besonderer Bedeutung dabei ist es, daß die auf der Rückseite aufgebrachte wasserfeste, an sich geschlossene Beschichtung eine geringere Dehnbarkeit als der Vliesträger in zumindest einer Richtung aufweist, so daß im verklebten Zustand des Pflasters auf der Haut durch die Eigenbewegungen der Haut und der damit ausgelösten Spannungen, welche die Zwischenschicht vom Vliesträger auf die äußere duroplastische Schicht überträgt, diese in feinsten Haarrissen aufreißt. Dadurch erhält

2

das Pflaster die für die Hautatmung notwendige und besonders kritische Luftdurchlässigkeit, behält aber gleichzeitig seine Wasserundurchlässigkeit.

Der Vlies-Trägerstreifen muß eine gewisse Loch-Struktur mit einer Größe der Poren von 0,5 bis 2 mm Durchmesser aufweisen, weil nur an diesen Lochstellen die Haarrisse in ihrer Wirkung durch die sonst vorhandene Vliesstruktur nicht behindert werden.

Bei einer geringeren Porengröße des Vlieses wäre eine dünnere Beschichtung erforderlich, um mit den bei der normalen Bewegung der verklebten Haut auftretenden Kräften eine Porosität in der Lackschicht zu erzielen. Eine dünnere Beschichtung als etwa 5 µm würde aber nicht mehr die geforderte Wasserfestigkeit gewährleisten. Größere Poren als etwa 2 mm würden dagegen ebenfalls zu keinem brauchbaren Produkt mehr führen.

Vorzugsweise besteht das Vlies aus Polyesterfasern, welche ohne Bindemittel nach dem sog. »Splunlaced«-Verfahren mit Hilfe von feinen Wasserstrahlen verfestigt worden sind. Bei diesem Verfahren können Faservliese, die auf trockenem oder nassem Wege hergestellt worden sind, eingesetzt werden, und die Verfestigung erfolgt in der Weise, daß das Faservlies beispielsweise auf einem Drahtsieb oder einer perforierten Platte, deren Lochmusterung sich später im fertigen Vlies wieder zeigt, im Abstand von wenigen Zentimetern kontinuierlich unter mehreren Düsensätzen durchgeführt wird. Aus diesen Düsenöffnungen wird Wasser unter hohem Druck, der an den Öffnungen zwischen 14 und 70 kp/cm$^2$ liegen kann, hindurchgeführt. Die auf das Vlies aufprallenden Wasserstrahlen üben eine ähnliche Wirkung aus wie bei der Nadelfilztechnik, d. h. einzelne Fasern werden durch den Flüssigkeitsstrahl nach unten gezogen und infolge der turbulenten Wasserströmung innerhalb der Vliesbahn miteinander verknäuelt und verflochten.

Als duroplastische Schicht eignen sich vor allem hochvernetzte, mit Butanol verätherte Harnstoff-Formaldehyd-Harze oder auch Lacke auf Basis von Melaminharzen gegebenenfalls in Kombination mit Alkydharzen, die sich durch eine besondere Alterungsbeständigkeit und Stabilität auszeichnen. Die Dicke der Schicht sollte nur etwa 5—10 µm, vorzugsweise etwa 6 µm betragen, da der Lack in dieser Schichtstärke die gewünschte Wasserundurchlässigkeit bewirkt und durch die normale Bewegung der Haut zu feinen Haarrissen aufreißt, ohne das elastische und schmiegsame Verhalten des Vlieses zu beeinträchtigen.

Zur Bildung der elastischen Zwischenschicht, welche die duroplastische Schicht mit dem Vlies verbindet, lassen sich in erster Linie solche Polymerzusammensetzungen verwenden, welche erst nach Ausstreichen auf dem Trägermaterial zum endgültigen elastischen, nichtklebrigen Zustand ausreagieren und dabei noch eine gute Verbindung sowohl mit dem Vlies als auch mit der duroplastischen Schicht einzugehen vermögen. Auf diese Weise wird ein widerstandsfähiges Verbundprodukt erhalten. Vorzugsweise eignen sich für diesen Zweck polyurethanbildende Gemische, welche in situ zum Endprodukt reagieren.

Die Stärke dieser Zwischenschicht darf nur wenige µm, etwa 1—3 µm, vorzugsweise 1—2 µm betragen, um die für ein Pflaster erforderliche Porosität aufzuweisen. Eine derartig dünne Schicht reißt in Verbindung mit dem Vlies von selbst in feinen Poren auf.

Der Vliesträger ist auf seiner der Haut zugewandten Seite mit einer in bekannter Weise, vorzugsweise gemäß DBP 1 569 901, mikroporös aufgetragenen Selbstklebeschicht versehen, die durch die poröse Struktur des Vlieses noch zusätzlich zu einer Vielzahl größerer, wahrnehmbarer Poren aufgerissen ist.

Als reizarme und gut hautverträgliche Klebemassen haben sich insbesondere solche aus Polyacrylsäureestern oder Acrylsäureester-Copolymerisaten bewährt.

Der erfindungsgemäße Klebestreifen, der gegebenenfalls hautfarben eingefärbt sein kann, kann sowohl als Heftpflaster wie auch als Trägermaterial für Wundschnellverbände verwendet werden. Für diesen Zweck wird vorzugsweise ein querelastisch ausgebildetes Vlies verwendet, so daß der daraus hergestellte Wundverband ebenfalls quer zu seiner Längsrichtung elastisch dehnbar ist und sich dadurch besonders gut den Bewegungen der Haut anpassen kann, ohne sich abzulösen.

Die Wundauflage kann aus einem Gewebe, Gewirke, Faservlies oder Schaumstoff bestehen. Insbesondere sind solche Materialien geeignet, die unter dem Einfluß der Wundsekrete nicht mit der Wundoberfläche verkleben sowie gut saugfähig und luftdurchlässig sind. Vorzugsweise werden Gewebe oder Gewirke verwendet mit abwechselnd schrumpfenden und nichtschrumpfenden oder hochgedrehten Fäden, welche sich bei Einwirkung von Feuchtigkeit von der Wundoberfläche abheben.

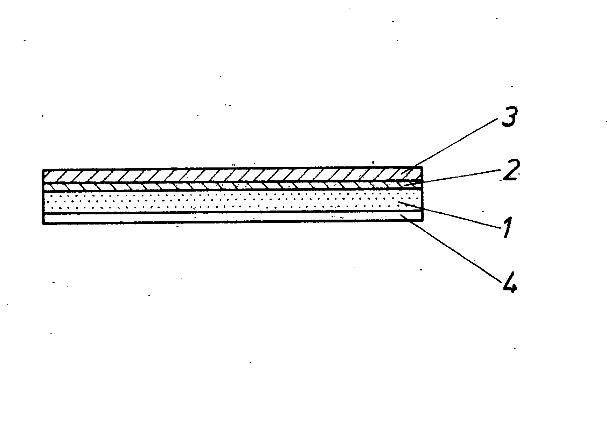

Die Abbildung zeigt den erfindungsgemäßen Klebestreifen im Querschnitt, wobei 1 den Vliesträger, 2 die elastische Zwischenschicht, 3 die duroplastische Deckschicht und 4 die Selbstklebeschicht darstellen.

Die Herstellung des Klebestreifens soll durch das folgende Ausführungsbeispiel beispielsweise erläutert werden.

Beispiel

Auf eine 25 µm starke Polyesterfolie wird mit einem Walzauftragswerk ein lösungsmittelhaltiger Harnstoff-Formaldehyd-Lack in einer Schichtstärke von 6 g/m$^2$ Trockengewicht aufgetragen. Der Lack besteht aus 77,6 Gewichtsteilen eines mit Butanol verätherten Harnstoff-Formaldehydharzes mit einer

3

Säurezahl von 15—20 und 21,4 Gewichtsteilen eines mit Butanol verätherten Harnstoff-Formaldehyd-Harzes mit einer Säurezahl von 6—10 und 1,0 Gewichtsteile p-Toluolsulfonsäure, gelöst in einem Gemisch aus Butanol/Isopropanol/Aceton. Nach der Entfernung der Lösungsmittel wird die Lackschicht in einem Trockenkanal bei Temperaturen zwischen 120 und 150° C vernetzt.

Im nächsten Arbeitsgang wird auf die voll vernetzte Lackschicht wiederum mit einem Walzenauftragswerk ein lösungsmittelhaltiger 2 Komponenten-Polyurethanklebstoff in einer so geringen Schichtstärke aufgetragen, daß sich kein zusammenhängender Film im getrockneten Zustand bilden kann. Bei einer Menge von 1—2 g/m$^2$ ist diese Forderung erfüllt.

Als Basiskomponente wird ein Prepolymer mit endständigen Isocyanatgruppen und als Vernetzerkomponente eine hydroxylhaltige Verbindung (handelsüblicher Härter auf Basis zweiwertiger Alkohole) eingesetzt.

Auf die noch klebrige Kaschiermasse wird ein nach dem »Spunlaced«-Verfahren hergestelltes besonders hautfreundliches Polyester-Vlies, das ein Lochmuster ähnlich einer Perforation besitzt, aufkaschiert und das Verbundprodukt im Trockenkanal auf etwa 80—100° C erhitzt, so daß eine Verankerung auf dem Vliesträger erfolgt.

Nach diesem Arbeitsgang wird die als Hilfsträger verwendete Polyesterfolie einkaschiert und man erhält den wasserfest beschichteten Vliesträger, aus dem das Pflaster durch geeignete Beschichtung mit einer Selbstklebemasse hergestellt wird.

Dieser Beschichtungsvorgang wird gemäß der Lehre des DBP 1 569 901 durchgeführt.

Hierzu wird auf einem mit einer ausgehärteten Silikonbeschichtung versehenen Papierträger eine viskoelastische Selbstklebemasse, die durch Copolymerisation von 490 Gewichtsteilen 2-Äthylhexylacrylat, 490 Gewichtsteilen n-Butylacrylat und 20 Gewichtsteilen Glycidylmethacrylat erhalten wurde, aus einer Lösung in einem Aceton-Benzin-Gemisch in einer solchen Menge aufgetragen, daß nach dem Trocknen eine Schichtstärke von ca. 30 g/m$^2$ erzielt wird. Der Trocknungsvorgang wird durch rasches Verdampfen des Lösungsmittels so durchgeführt, daß in der Klebemasse eine Vielzahl von Bläschen entstehen, die einen Durchmesser von 5—15 µm aufweisen. Auf die getrocknete Selbstklebemasse wird dann das wasserfest beschichtete Vlies unter Anwendung von Druck aufkaschiert, wobei die Bläschen zerstört werden und sich poröse bis mikroporöse Poren in der Selbstklebeschicht bilden. Die wasserfeste Beschichtung auf der anderen Seite des Vlieses wird hierbei nicht beeinträchtigt, da diese auf Grund des erfindungsgemäßen Aufbaus nur auf Zug und nicht auf Druckspannung reagiert.

Das Verbundprodukt wird anschließend in einem Trockenkanal bei ca. 130° C erhitzt, um einerseits die Selbstklebemasse zu vernetzen und andererseits eine Verankerung derselben mit dem Vlies durch die zwischen den Clycidylmethacrylatgruppen des Klebers und den Polyestergruppen des Vlieses ablaufende Reaktion zu erreichen.

Bei der Weiterverarbeitung zu einem Wundschnellverband wird das als Zwischenträger benutzte Trennpapier entfernt, die saugende Wundauflage aufgebracht und das fertige Pflaster erneut mit silikonisiertem Papier oder einer klebstoffabweisenden Folie eingedeckt und für den Verkauf konfektioniert. Gegebenenfalls können die Wundverbände nach dem Verpacken beispielsweise einer $\gamma$-Bestrahlung unterworfen und auf diese Weise sterilisiert werden.

Das auf der Haut verklebte Pflaster ist hervorragend atmungsaktiv, bedingt durch die gute Luftdurchlässigkeit, die in der Klebemasse durch das Beschichtungsverfahren und in der wasserdichten Beschichtung des porösen Vlieses durch die auf Grund der Bewegungen der Haut erzeugten Spannungen hervorgerufenen Haarrisse erzeugt wird. Gewünschtenfalls können diese feinen Risse durch Ausüben einer leichten Zugspannung beim Aufbringen des Pflasters noch vergrößert werden.

Zum Nachweis dieser Haarrisse wurde das wasserfest beschichtete Vlies mit und ohne Selbstkleber verschieden stark gedehnt und sowohl an den Ausgangsprodukten als auch an den gedehnten Produkten die Luftdurchlässigkeit (mit dem Gurley-Densometer) und Wasserdampfdurchlässigkeit gemessen. Die Ergebnisse aus der folgenden Tabelle bestätigen den Einfluß der durch Zugspannungen hervorgerufenen Dehnungen auf die Luft- und Wasserdampfdurchlässigkeit.

| Dehnung in % | Luftdurchlässigkeit: cm$^3$/cm$^2$ · sec | | Wasserdampfdurchlässigkeit: g H$_2$O/m$^2$ · 24 h | |
|---|---|---|---|---|
| | Vlies mit wasserfester Beschichtung | Vlies mit wasserfester Beschichtung und Selbstklebeschicht | Vlies mit wasserfester Beschichtung | Vlies mit wasserfester Beschichtung und Selbstklebeschicht |
| 0 | 0 | 0 | 505 | 424 |
| 10 | 3,1 | 0,55 | 750 | 490 |
| 20 | 12,9 | 3,1 | 978 | 552 |
| 40 | >30 | 7,7 | 1215 | 792 |

## Patentansprüche

1. Wasserfester Klebestreifen für medizinische Zwecke aus einem einseitig mit einer Selbstklebemasse versehenen Trägerstreifen auf Basis eines porösen, mindestens in einer Richtung elastischen dünnen Faservlieses, dadurch gekennzeichnet, daß das Faservlies Poren einer Größe von 0,5—2 mm aufweist und auf seiner nichtklebenden Rückseite mit einer ca. 5—10 µm starken duroplastischen, wasserundurchlässigen Lackschicht versehen ist, welche mittels einer porösen ca. 1—3 µm starken elastischen Zwischenschicht mit der Vliesoberfläche verbunden ist.

2. Klebestreifen gemäß Anspruch 1, dadurch gekennzeichnet, daß die auf der Rückseite aufgebrachte, wasserfeste, geschlossene Beschichtung eine geringere Dehnbarkeit als der Vliesträger in zumindest einer Richtung aufweist und unter Einwirkung von Dehnungsvorgängen Spannungsrißbildung in der Beschichtung auftritt.

3. Klebestreifen gemäß Anspruch 1 und 2, dadurch gekennzeichnet, daß der Vliesträger aus einem bindemittelfreien, porösen Polyesterfaser-Vlies besteht.

4. Klebestreifen gemäß Anspruch 1 bis 3, dadurch gekennzeichnet, daß die duroplastische Schicht aus einem oder mehreren hochvernetzten, mit Butanol verätherten Harnstoff-Formaldehyd-Harzen besteht.

5. Klebestreifen gemäß Anspruch 1 bis 4, dadurch gekennzeichnet, daß die elastische Zwischenschicht aus einem Polyurethan besteht.

6. Klebestreifen gemäß Anspruch 1 bis 5, dadurch gekennzeichnet, daß die Klebeschicht aus einer Selbstklebemasse auf Basis von Polyacrylsäureestern oder Acrylsäureester-Copolymerisaten besteht und porös ausgebildet ist.

7. Klebestreifen gemäß den Ansprüchen 1 bis 6, dadurch gekennzeichnet, daß er quer zu seiner Längsrichtung elastisch dehnbar ausgebildet ist und eine Wundauflage auf ihm aufgebracht ist, wobei mindestens an zwei gegenüberliegenden Seiten der Auflage eine klebende Zone freibleibt.

## Claims

1. Water-resistant adhesive strip for medical purposes, comprising a carrier strip, based on a porous thin fibre fleece which is elastic in at least one direction, the carrier strip being provided with a self-adhesive composition on one side, characterized in that the fibre fleece has pores of size 0.5—2 mm and is provided, on its non-adhesive rear face, with an approximately 5—10 µm thick thermoset water-impermeable lacquer coating bonded to the fleece surface by means of a porous approximately 1—3 µm thick elastic intermediate layer.

2. Adhesive strip according to claim 1, characterized in that the water-resistant, continuous coating applied to the rear face has a lower extensibility than the fleece carrier, in at least one direction, and that under the action of stretching processes stress crack formation occurs in the coating.

3. Adhesive strip according to claim 1 and 2, characterized in that the fleece carrier consists of a binderfree porous polyester fibre fleece.

4. Adhesive strip according to claims 1 to 3, characterized in that the thermoset coating consists of one or more highly crosslinked butanol-etherified urea-formaldehyde resins.

5. Adhesive strip according to claims 1 to 4, characterized in that the elastic intermediate layer consists of a polyurethane.

6. Adhesive strip according to claims 1 to 5, characterized in that the adhesive layer consists of a self-adhesive composition based on polyacrylic acid esters or acrylic acid ester copolymers and has a porous strukture.

7. Adhesive strip according to claims 1 to 6, characterized in that it is elastically stretchable transversely to its lengthwise direction and that a wound covering is applied to it, an adhesive zone being left free at least on two opposite sides of the covering.

## Revendications

1. Bande adhésive hydrofuge à usage médical, cette bande étant constituée d'une bande support pourvue d'une masse auto-adhésive sur une face et à base d'un mince voile de fibres poreux élastique dans au moins une direction, caractérisée en ce que le voile de fibres comporte des pores d'une dimension de 0,5 à 2 mm tandis que, sur sa face dorsale non adhésive, il est pourvu d'une couche de vernis duroplaste imperméable à l'eau d'une épaisseur d'environ 5—10 µm, cette couche étant assemblée à la surface du voile au moyen d'une couche intermédiaire poreuse élastique d'une épaisseur d'environ 1—3 µm.

2. Bande adhésive suivant la revendication 1, caractérisée en ce que l'enduit fermé hydrofuge appliqué sur la face dorsale a une plus faible extensibilité que le support de voile dans au moins une direction tandis que, sous l'influence de processus d'extension, il se forme des fissures de tension dans l'enduit.

3. Bande adhésive suivant les revendications 1 et 2, caractérisée en ce que le support de voile est constitué d'un voile poreux de fibres de polyester, exempt d'agent liant.

4. Bande adhésive suivant les revendications 1 à 3, caractérisée en ce que la couche duroplaste est constituée d'une ou de plusieurs résines d'urée-formaldéhyde fortement réticulées et éthérifiées avec du butanol.

5. Bande adhésive suivant les revendications 1 à 4, caractérisée en ce que la couche intermédiaire élastique est constituée d'un polyuréthane.

6. Bande adhésive suivant les revendications 1 à 5, caractérisée en ce que la couche adhésive est constituée d'une masse auto-adhésive à base d'esters d'acide polyacrylique ou de copolyméres d'esters d'acide acrylique, tandis qu'elle a une structure poreuse.

7. Bande adhésive suivant les revendications 1 à 6, caractérisée en ce qu'elle a une structure élastiquement extensible transversalement à son sens longitudinal, tandis qu'un pansement médical y est appliqué, une zone collante restant libre au moins sur deux côtés opposés du pansement.